# EUROPEAN PATENT SPECIFICATION

(11) **EP 1 636 383 B1**
(45) Date of publication and mention of the grant of the patent: **23.11.2016**
(21) Application number: 04773939.6
(22) Date of filing: 16.06.2004
(51) Int. Cl.: C12Q 1/68

(54) **COMPOSITION FOR SCREENING ANTI-HYPERTENSION DRUG COMPRISING MAMMAL TCTP GENE OR ITS PROTEIN PRODUCT, AND METHOD FOR SCREENING ANTI-HYPERTENSION DRUG USING SAID COMPOSITION**
DAS TCTP-GEN AUS SÄUGERN ODER DESSEN PROTEINPRODUKT UMFASSENDE ZUSAMMENSETZUNG ZUM SCREENING EINES BLUTHOCHDRUCKMITTELS UND VERFAHREN ZUM SCREENING AUF EIN BLUTHOCHDRUCKMITTEL UNTER VERWENDUNG DER ZUSAMMENSETZUNG
COMPOSITION DESTINEE A L'IDENTIFICATION PAR CRIBLAGE D'UN MEDICAMENT ANTIHYPERTENSEUR, CONTENANT UN GENE TCTP DE MAMMIFERE OU SON PRODUIT PROTEIQUE, ET PROCEDE D'IDENTIFICATION D'UN MEDICAMENT ANTIHYPERTENSEUR AU MOYEN DE LADITE COMPOSITION

(30) Priority: 21.06.2003 KR 2003040519; 15.06.2004 KR 2004043909
(43) Date of publication of application: 22.03.2006
(73) Proprietor: Ewha University Industry Collaboration Foundation, Seodaemoon-ku Seoul 120-750 (KR)
(72) Inventor: LEE, Kyung-Lim, Div. Molecular Life Sciences, Seodaemoon-ku, Seoul 120-750 (KR); OH, Goo-Taeg, Daejon 305-755 (KR); CHO, Myeong-Chan, Circulatory Chungbuk Uni. Hosp., Chungju-city 361-711 (KR); KIM, Min-Jeong, Suwon, Kyunggi-do 440-320 (KR)
(74) Representative: Winter, Brandl, Fürniss, Hübner, Röss, Kaiser, Polte - Partnerschaft mbB
(86) International application number: PCT/KR2004/001435
(87) International publication number: WO 2004/113572

(56) References cited:
- WO-A1-02/22170
- WO-A2-02/084301
- US-A1- 2002 177 692
- US-B1- 6 358 738
- JUNG J ET AL: "Translationally controlled tumor protein interacts with the third cytoplasmic domain of Na,K-ATPase alpha subunit and inhibits the pump activity in HeLa cells" JOURNAL OF BIOLOGICAL CHEMISTRY, AMERICAN SOCIETY OF BIOLOCHEMICAL BIOLOGISTS, BIRMINGHAM,, US, vol. 279, no. 48, 26 November 2004 (2004-11-26), pages 49868-49875, XP002374198 ISSN: 0021-9258
- KIM M J ET AL: "SYSTEMIC HYPERTENSION IN TRANSGENIC MICE OVEREXPRESSING TRANSLATIONALLY CONTROLLED TUMOR PROTEIN" FEBS JOURNAL, BLACKWELL PUBLISHING, LONDON, GB, vol. 272, no. SUPPL 1, 2 July 2005 (2005-07-02), pages 193-194, XP009083725 ISSN: 1742-464X
- KAKUTURU ET AL.: 'Cloning and Characterization of a Calcium-binding, Histamine-releasing Protein from Schistosoma mansoni' J. BIOL. CHEM. vol. 277, no. 4, August 2002, pages 31207 - 31213, XP003016364
- CHUNG S. ET AL.: 'Expression of translationally controlled tumor protein mRNA in human colon cancer' CANCER LETTERS vol. 156, August 2000, pages 185 - 190, XP002199184

## Description

### Background of the Invention

### Technical Field

The present invention relates to a composition for screening antihypertensive drugs, and a method for screening antihypertensive agents using the composition.

### Background Art

Translationally controlled tumor protein (TCTP) is a protein known to have histamine-releasing function, as first reported by Thueson et al in a paper "J Immunol., 123(2):626-32, (1979)". The paper describes that a histamine-releasing factor (HRF) has the activity to release histamine from cultured human mononuclear cells.

HRF which is a product from activated immune cells was defined as a substance that induce histamine release by interaction with basophils and mast cells. Then, it was classified into two categories. One requires IgE on cell surfaces upon histamine liberation, and the other acts in an IgE-independent manner. Of them, the IgE-dependent histamine-releasing factor (HRF) is believed to stimulate histamine release from basophils without antigens, thus playing an important role in the development of late allergic inflammatory responses.

In mice, the HRF protein is termed P21 meaning a 21-kDa polypeptide, and in human beings, it is known as P23. Since it was established in 1988 that the expression of HRF is regulated at the translation level by serum and regulated at the transcription level by lead, copper, cadmium, etc., HRF has been named "translationally controlled tumor protein (TCTP)".

P21 was reported to be an acidic peptide which is a 2D-PAGE reference map and has an isoelectric point of 4.9 and a molecular weight of 24 kDa. Then, by MacDonald et al. (Science, 269:688-690, 1995), HRF was isolated and purified from the lymphocytes of atopic patient and the biological fluids of allergic patient, and by its amino-terminal sequencing, its was found to be the same protein as TCTP which has been known as a growth-related protein.

TCTP has been known as a tumor-specific protein until 1980s, and its synthesis has been thought to be associated with the proliferative stage of tumors. However, it was reported that TCTP is found in all normal cells excluding kidney and renal cell carcinomas and shows high homology in almost all species.

TCTP is thus thought to be a housekeeping protein which plays a very important role in cells and is expressed at a constant level, but its definite functions are not yet known

In the patent publication WO 0222170 A1, HRF is described as a factor which decreases insulin secretion from pancreatic cells and appears therefore useful in a medical indication caused by excess of insulin such as type 2 diabetes or hypertension.

As disclosed in Korean Patent Application KR 10 2001 109481 A, the present inventors previously found that HRF known to be the same protein as TCTP interacts with the third cytoplasmic loop of the subunits of the Na/K-ATPase pump.

TCTP is a protein obtained by performing yeast two-hybrid screens from a rat skeletal muscle library using the third cytoplasmic loop of the Na/K-ATPase pump as bait. Its interaction is not isoform specific, and it interacts only with the third cytoplasmic loop of the five cytoplasmic loops of the Na/K- ATPase pump. This interaction was confirmed again by biacore assay, coimmunoprecipitation, and confocal microscope. At this time, TCTP was shown to act as a cytoplasmic repressor of the Na/K-ATPase pump.

The Na/K-ATPase pump is a membrane protein which is present on the cellular membrane and consists of two subunits, i.e., a 112-kDa subunit and a 55-kDa subunit. The most important function of the Na/K-ATPase pump is to maintain the concentration of K⁺ ions in cells at a high level by ion transport across the membrane, and to increase the concentration of Na⁺ ions outside cells, thus regulating the intracellular Na⁺ and K⁺ ion concentrations.

Moreover, the Na/K-ATPase pump also acts as a signaling molecule in protein-protein interactions. Substances that inhibit the ion concentration regulatory function of the Na/K-ATPase pump include glycoside ouabain, digoxin, digitoxin and the like, and the long-term administration of such glycosides results in the contraction of blood vessels and the increase of blood pressure. Presumably, this is attributed to an increase in the intracellular Na⁺ and Ca²⁺ concentrations caused by a reduction in the function of the Na/K-ATPase pump.

In connection with this, a hypothesis has been proposed that a endogenous ouabain-like factor suppressing the activity of the Na/K-ATPase pump is present in cells and contributes to cause hypertension and heart hypertrophy. Recently, experimental evidence was also proposed that the repression of the Na/K-ATPase pump could activate NF-kB by cross talk with EGFR so as to cause heart hypertrophy (Haas, M., Askari, A., and Xie, Z., J. Biol. Chem., 275:27832-27837, 2000).

As a result, it is expected that inhibitors of the Na/K-ATPase pump will cause hypertension and heart hypertrophy, and effective screening of substances capable of inhibiting the activity of these inhibitors will be very important in the development of antihypertensive agents.

Accordingly, the present inventors have confirmed that TCTP causes hypertension and heart hypertrophy by inhibiting the activity of the Na/K-ATPase pump, and developed a composition and method for screening antihypertensive drugs using TCTP, thereby perfecting the present invention.

### Disclosure of the Invention

An object of the present invention is to provide a composition for use in screening antihypertensive drugs, which contains a mammalian TCTP or a protein expressed therefrom, as well as a method for screening antihypertensive drugs using the composition.

The present invention provides the use of a composition for screening antihypertensive drugs, which contains a mammalian TCTP gene, wherein the antihypertensive drugs inhibit an expression of the mammalian TCTP gene.

The TCTP gene contained in the composition is a gene found in almost all species of mammalian cells and has either a base sequence of SEQ ID NO: 1 or a base sequence having one or more disruption, deletion, insertion, point, substitution, nonsense, missense, polymorphism or rearrangement mutations in the base sequence of SEQ ID NO: 1.

In one aspect, the present invention provides the use of a composition for screening antihypertensive drugs, which contains a mammalian TCTP protein, wherein the antihypertensive drugs inhibit the function of the mammalian TCTP protein to act as an intracellular inhibitor of the Na/K ATPase pump.

The TCTP protein contained in the composition is a housekeeping protein which is expressed in almost all species of mammalian cells at a constant level. This protein has an amino acid sequence of SEQ ID NO: 2, or is a polypeptide fragment which can be expressed from a gene of a base sequence having one or more disruption, deletion, insertion, point, substitution, nonsense, missense, polymorphism or rearrangement mutations in the base sequence of SEQ ID NO: 1 and shows physiological activity equal to that of TCTP.

In another aspect, the present invention provides a method for screening antihypertensive drugs, which uses the TCTP gene-containing composition as a target substance.

The present invention provides a method for screening antihypertensive drugs, the method comprising the steps of: contacting the TCTP gene-containing composition with a test substance, and examining the reaction between the composition and the test material so as to determine if the test substance exhibits the activity to inhibit the expression of the gene contained in the composition.

In another embodiment, the present invention provides a method for screening antihypertensive drugs, which uses the TCTP protein-containing composition as a target substance.

The present invention provides a method for screening antihypertensive drugs, the method comprising the steps of: contacting the TCTP protein-containing composition with a test substance; and examining the reaction between the composition and the test substance so as to determine if the test substance exhibits the activity to inhibit the function of the protein contained in the composition.

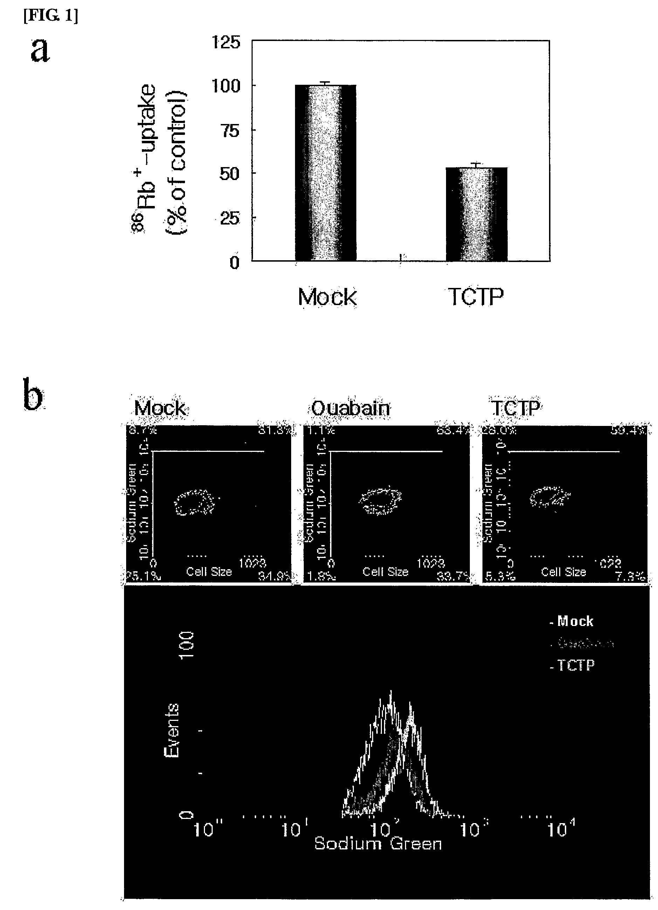

TCTP, which is a protein binding to the large cytoplasmic loop of the Na/K APTase pump, acts an intracellular inhibitor of the Na/K APTase pump (FIG. 1), thus causing hypertension and heart hypertrophy.

A mechanism by which TCTP causes hypertension and heart hypertrophy seems to be associated with a phenomenon where the inhibition of the Na/K APTase pump function influences the response and contraction of vascular smooth muscles and heart muscles so as to cause hypertension.

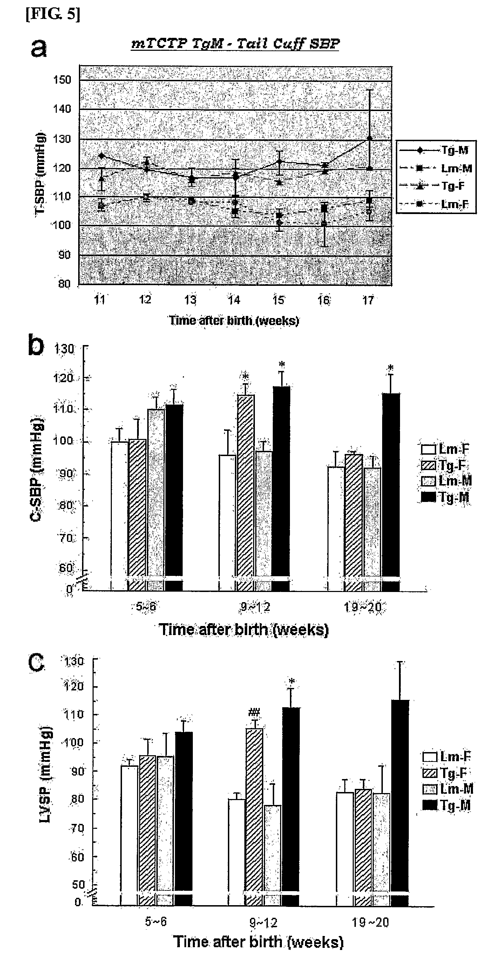

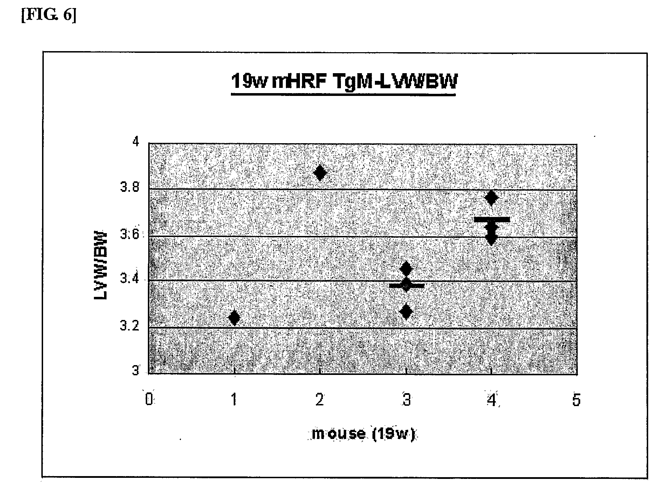

Mice which had been transformed to overexpress TCTP show phenotypes of remarkable hypertension symptoms (FIG. 5) and heart hypertrophy (FIG. 6) as compared to normal mice.

Accordingly, in the present invention, antihypertensive drugs are screened using a TCTP gene or a protein expressed therefrom, as a target substance.

The inventive screening method comprises the steps of contacting the composition with a test substance, and examining the reaction between the composition and the test substance so as to determine if the test substance exhibits the activity to inhibit either the expression of the gene or the function of the protein.

In the inventive screening method, the reaction between the TCTP gene-containing composition and the test substance can be examined by conventional methods which are used in confirming that DNA-DNA, DNA-RNA, and DNA-protein reactions occurred.

Examples of the methods which can be used to examine the reaction include: a hybridization test to examine the binding between the gene and the test substance *in vitro;* a method of measuring the expression level of the gene by Northern blot analysis after reaction between mammalian cells and the test substance; and a method where the gene linked with a reporter gene is introduced into cells and then reacted with the test substance, and the expression level of the reporter protein is measured.

In this case, in addition to the TCTP gene, the composition may contain distilled water or buffer which maintains the structure of nucleic acid stable.

In the inventive screening method, the reaction between the TCTP protein-containing composition and the test substance can be examined by conventional methods which are used in confirming that protein-protein reaction occurred.

Examples of the methods which can be used to examine the reaction include: a method of measuring the activity of the TCTP gene or protein after reacting the TCTP gene or protein with the test substance; a yeast two-hybrid method; screening of a phage-displayed peptide clone binding to the TCTP protein; high throughput screening (HTS) using natural and chemical library; drug hit HTS; cell-based screening; and screening methods using a DNA array.

In this case, in addition to the protein expressed from the TCTP gene, the composition may contain buffer or reaction solution which maintains the structure or physiological activity of the protein stable. Furthermore, for *in vivo* experiments, the composition may contain either a cell expressing the protein, or a cell containing a plasmid which expresses the protein under the presence of a promoter capable of regulating transcriptional level.

In the inventive screening method, the test substances may be individual nucleic acids, proteins, or other extracts or natural substances, which are either presumed to have the possibilities as antihypertensive drugs according to a conventional selection method or randomly selected.

Antihypertensive drug candidates obtained by the inventive screening method will act as leading compounds in a subsequent step for developing antihypertensive drugs. The structure of the leading substances can be modified and optimized such that it can exhibit an inhibitory effect against the TCTP gene or the protein expressed therefrom. This will result in the development of new antihypertensive drugs.

Since the drugs thus obtained will exhibit a partial or complete inhibitory effect against the mammalian TCTP gene or the protein expressed therefrom, they can inhibit hypertension, heart hypertrophy, and other diseases, which are caused by the TCTP gene or the protein expressed therefrom.

As a result, the composition and the screening method using the same will be useful for the investigation and development of antihypertensive drugs against pathogenic bacteria including those derived from mammals.

In another aspect, the present invention uses transgenic mice which contain the mammalian TCTP gene at somatic and generative cells by the introduction of the mammalian TCTP gene at the embryonic stage, and thus show phenotypes of hypertension and heart hypertrophy by the overexpression of a TCTP protein from the TCTP gene.

In the transgenic mice, the TCTP gene may have either the base sequence of SEQ ID NO: 1 or a base sequence having one or more disruption, deletion, insertion, point, substitution, nonsense, missense, polymorphism or rearrangement mutations in the base sequence of SEQ ID NO: 1.

For introduction into the embryos of the transgenic mice, the TCTP gene is inserted into a transformation vector for mice. Preferably, the TCTP gene is inserted into a transformation vector DNA containing a cytomegalovirus enhancer (CMV-IE), a chicken beta-actin promoter and a rabbit beta-globin poly A-tail.

The embryos which can be used to produce the transgenic mice are embryos derived from inbred or hybrid mice. Preferably, C57BL/6N inbred mice or C57BL/6J + CBA/N hybrid mice can be used.

The embryos of the TCTP-overexpressing transgenic mice were deposited under accession number KCTC 10640BP on May 21, 2004 with the Korean Collection for Type Cultures (KCTC), Korean Research Institute of Bioscience and Biotechnology.

The TCTP-overexpressing transgenic mice can be produced by a method comprising the steps of: 1) inserting a mammalian TCTP gene into a transformation vector to produce a recombinant gene construct for transformation; 2) microinjecting the recombinant gene construct from the step 1) into the male pronucleus of the embryo of a mouse; 3) implanting the microinjected embryo into a surrogate mother mouse; and 4) selecting TCTP-overexpressing transgenic mice from the progeny of the surrogate mother mouse, by confirming that the transgenic mice have the TCTP gene inserted into a genomic DNA, express a TCTP protein and show a phenotype of hypertension or heart hypertrophy.

In the step 1), the TCTP gene has either the base sequence of SEQ ID NO: 1 or a base sequence having one or more disruption, deletion, insertion, point, substitution, nonsense, missense, polymorphism or rearrangement mutations in the base sequence of SEQ ID NO: 1.

Moreover, the transformation vector which is used in the step 1) may be a vector for transforming mice, which is known in the art. A promoter in the vector may be either a promoter inducing expression in total tissue or a promoter inducing tissue-specific expression, and contains not only an enhancer for activating the promoter, and a poly A-tail following a terminator. Preferably, a vector containing a cytomegalovirus enhancer (CMV-IE), a chicken beta-actin promoter and a rabbit beta-globin poly A-tail may be used.

The mammalian TCTP gene is inserted into the vector for transforming mice so as to prepare a recombinant gene construct for transformation. Then, the DNA base sequence of the recombinant gene construct is analyzed, produced at large amounts, purified in various steps, and diluted in a microinjection solution to a concentration suitable for microinjection.

In the step 2), the embryos to be microinjected with the TCTP gene are prepared from inbred or hybrid mice, and the recombinant gene construct is microinjected into the male pronuclei of the prepared mouse embryos using micromanipulator. The insertion of the gene into the male pronuclei is confirmed by the expansion of the male pronucleus. The embryos microinjected with the TCTP gene are cultured to the two-cell stage in a CO₂ incubator.

In the step 3), the surrogate mother mouse to be implanted with the embryo microinjected with the foreign gene in the step 2) is prepared by mating an ICR female mouse with a vasectomized male mouse. Then, the embryo microinjected with the TCTP gene in the above step is implanted into the oviduct of the surrogate mother mouse.

In the step 4), progeny are born from the surrogate mother mouse after implantation with the embryo, and in order to confirm the introduction and expression of the TCTP gene in the born progeny, DNA and protein are extracted from the mouse tail. The TCTP-overexpressing transgenic mice are selected from the progeny, by confirming that the transgenic mice have the TCTP gene inserted into the mouse genomic DNA, express a TCTP protein and show a phenotype of hypertension or heart hypertrophy.

The method for producing transgenic mice can be performed by any known method (Ian J. Jackson; Catherine M. Abbott; Mouse genetics and transgenics: a practical approach, Oxford; New York: Oxford University Press, 2000) or a modification of the known method.

In another aspect, the present invention provides a method for screening antihypertensive drugs, which comprises administering test substances to the transgenic mice overexpressing TCTP; observing the extent of improvement in hypertension and heart hypertrophy symptoms in the animal; and screening test substances showing the improvement.

### Brief Description of Drawings

FIG. 1 shows the Na/K APTase pump inhibitory activity of TCTP contained in the screening composition used in the invention.
FIG. 2 shows a genetic map required for the production of transgenic mice which overexpress TCTP contained in the screening composition used in the invention.
FIG. 3 shows that a TCTP foreign gene is inserted into a genomic DNA extracted from the tail of TCTP-overexpressing transgenic mice.
FIG. 4 shows the overexpression of a TCTP gene in the internal organs of TCTP-overexpressing transgenic mice.
FIG. 5 shows hypertension symptoms in TCTP-overexpressing transgenic mice. FIG. 5a shows measurement results for tail systolic blood pressure in the transgenic mice, FIG. 5b shows measurement results for carotid systolic blood pressure in the transgenic mice, and FIG. 5c shows measurement results for left ventricular systolic blood pressure in the transgenic mice.
FIG. 6 shows the expression of heart hypertrophy in TCTP-overexpressing transgenic mice.

### Best Mode for Carrying Out the Invention

The present invention will hereinafter be described in further detail by examples. It is to be understood, however, that the scope of the present invention is not limited to or by these examples.

### Example 1: Examination of use of a composition for screening antihypertensive drugs

### 1) Examination of Na/K APTase pump inhibitory activity of TCTP protein contained in the composition

The Na/K APTase pump inhibitory activity of the TCTP protein contained in the composition was examined. As it was already known that TCTP interacts with the Na/K APTase pump, a test was performed to examine if the interaction has any effect on the activity of the Na/K APTase pump inhibitory activity.

RBL-2H3 cells were cultured at a suitable density and washed three times with Krebs-Ringer buffer (KRP, 140 mM NaCl, 5 mM KCl, 10 mM Na₂HPO₄, 1 mM MgSO₄, 1.4 mM CaCl₂, 2.5 mM glucose, pH 7.4). Subsequently, the cells were incubated at 37 °C for 15 minutes, and added with 0.1% BSA-containing KRP buffer containing 1mM ouabain.

To measure the transport of K⁺ ions caused by the Na/K APTase pump, cultivation was performed for 5-10 minutes using an ⁸⁶Rb⁺ (0.5 Ci/ml, NEN) tracer having high sensitivity to ouabain, and 10-20 µg/ml TCTP was added. Also, a control group to which TCTP had not been added was prepared. The uptake of ⁸⁶Rb⁺ and the consumption of K⁺ were measured and the results are shown in FIG. 1.

As shown in FIG. 1a, TCTP greatly reduced the activity of the Na/K APTase pump to 53.5%.

To confirm the above results again, FACS analysis was performed using a Na-sensitive dye.

As shown in FIG. 1b, TCTP increased the intracellular Na⁺ concentration, unlike the control group (Mock) where Na transport was smoothly made.

Accordingly, it can be found that TCTP contained in the composition inhibits the ion concentration regulatory activity of the Na/K APTase pump, and is a cytoplasmic inhibitor of the Na/K APTase pump.

### 2) Confirmation of hypertension and heart hypertrophy caused by the composition used in the invention.

In order to confirm that hypertension and heart hypertrophy symptoms are caused in cells which continuously overexpress TCTP contained in the composition, TCTP-overexpressing transgenic mice were constructed.

### 2-1) Construction of transgenic mice overexpressing TCTP

The TCTP-overexpressing transgenic mice were constructed from C57BL/6N inbred mice. In the construction process of the transgenic mice, animal tests, including the collection of mouse embryos, the microinjection of genes into the embryos, and the implantation of the embryos into surrogate mother mice, were performed in Macrogen, Inc., Korea.

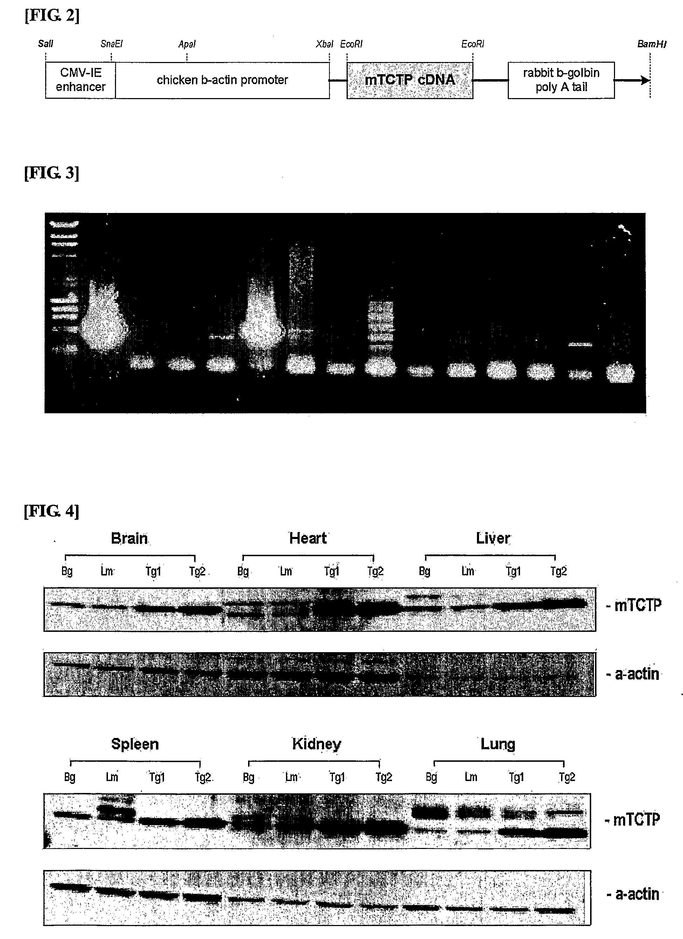

A genetic map used in the construction of the TCTP-overexpressing transgenic mice is shown in FIG. 2. This genetic map was designed to express TCTP in the total tissue of the transgenic mice. In the genetic map, a cytomegalovirus enhancer (CMV-IE) and a chicken beta-actin promoter, which had been provided by the Animal Laboratory, Korean Research Institute of Bioscience and Biotechnology, were used.

This gene construct was microinjected into C57BL/6N inbred mice by the following procedures.

First, TCTP cDNA was inserted into a vector DNA containing the cytomegalovirus enhancer and the chicken beta-actin promoter and then confirmed by DNA base sequencing.

The DNAs were collected at large amounts using a Qiagen DNA purification kit, and treated with restriction enzyme BamHI/SalI so as to make a DNA fragment. The DNA fragment was electrophoresed on low-melting agarose gel so as to obtain about 3-Kbp DNA fragment containing an enhancer, a promoter, TCTP cDNA and a rabbit beta-globin poly A-tail. Gel containing the DNA fragment was cut and placed in high salt buffer (20 mM Tris-HCl, 1.0 M NaCl, 1.0 mM EDTA, pH 7.4). The collected DNAs were finally purified using an Elutip-D (Schleicher & Schuell) column, dialyzed in microinjection solution (10mM Tris-HCl, 0.1 mM EDTA, pH 7.4) and controlled to a concentration of about 4-6 ng/µl. Then, 50 µl of the DNA solution was placed into each well and stored at -20 °C for use.

The TCTP gene was microinjected into the male pronucleus of the mouse embryo using a micromanipulator (Leitz, Germany), and whether the gene had been injected into the nucleus was confirmed by the expansion of the male pronucleus.

A surrogate mother mouse to be implanted with the embryo microinjected with the foreign gene was prepared by the following procedures. ICR female mice showing puberty in natural conditions were mated with vasectomized male mice before one day of embryo collection. In this step, the male mouse that had not been mated for at least 3 days was mated at 1:2 with the female mice, and on the next day morning, the vaginal smear of the female mice was examined and a plugged female mouse was used as a surrogate mother mouse.

The embryos microinjected with the TCTP gene were cultured in a CO₂ incubator to the two-cell stage, and then healthy embryos were selected and implanted. First, an anesthetic (Avertin) was injected into the abdominal cavity of the surrogate mother mice at the amount of 0.5 mg/10 g body weight to achieve the general anesthesia of the mice. The abdominal wall midline of the anesthetized mice was incised about 1 cm, the ovaries and oviducts were taken out, and the embryo was implanted into each of the oviducts. After confirming that the ampulla of the oviducts slightly swollen, the oviducts and ovaries were carefully placed again into the abdominal cavity, and the muscular layer and the outer skin were sutured to each other.

In order to confirm that the progeny born from the surrogate mother mice expresses the TCTP gene, the tail of the progeny was cut off about 0.5 cm, and a genomic DNA was extracted from the cut tail using a lysis buffer containing proteinase K and subjected to PCR to determine the germline transmission of the TCTP gene. Also, a protein was extracted from the internal organs of the mice, including brains, heart, liver, spleen, kidneys and lungs, and subjected to Western blotting to examine the expression of the TCTP protein.

As a result, TCTP transgenic mice confirmed to overexpress the TCTP gene were obtained from C57BL/6N inbred mice, and the embryos of the transgenic mice were deposited under accession number KCTC 10640BP on May 21, 2004 with the Korean Collection for Type Cultures (KCTC), Korean Research Institute of Bioscience and Biotechnology.

### 2-2 Construction of TCTP-overexpressing transgenic mice

Transgenic mice were constructed in the same manner as in Example 2-1 except that C57BL/6J + CBA/N hybrid mice were used.

PCR and Western blotting were performed on the TCTP-overexpressing transgenic mice constructed from the hybrid mice in the same manner as in Example 2-1, so as to examine whether the TCTP gene was inserted or not and whether the TCTP protein was expressed or not.

The results are shown in FIG. 3, an electrophoresis photograph. In FIG. 3, lane 2 is a positive control group, and the PCR product of the same position as that of the positive control group was observed at lane 6, suggesting that the TCTP gene was inserted into the progeny at the lane 6.

A protein was extracted from the internal organs of the mice, including brains, heart, liver, spleen, kidneys and lungs, and subjected to Western blotting. The results confirmed that the TCTP protein is expressed in all the internal organs of the transgenic mice.

### 2-3) Confirmation of hypertension and heart hypertrophy caused in TCTP-overexpressing transgenic mice

On each of the male and female of the TCTP-overexpressing transgenic hybrid mice constructed in Example 2-2), the measurement of systolic blood pressure was performed by the tail-cuff method, and the measurements of carotid systolic blood pressure and left ventricular systolic blood pressure were performed by a direct method using a catheter. Also, left ventricular hypertrophy was measured to examine whether heart hypertrophy occurred or not.

The tail systolic blood pressure (T-SBP) was measured using a computerized tail-cuff system (BA-98A system, Softron Co). The blood pressure measurement on the mice was initiated from 11 weeks after the birth of the mice, and repeated at one-week intervals on the same mice. In this case, the mice were divided into four groups consisting of male and female control mouse groups, and male and female transgenic mouse groups, and at least three mice were selected from each group and measured for blood pressure.

Furthermore, on each of the male and female of 5-6-week, 9-12-week, and 19-20-week-old transgenic mice, a 1.4 French high-fidelity micromanometer catheter was inserted directly into the carotid of the mice, and the carotid systolic blood pressure (C-SBP) was measured. The catheter was pushed in the left ventricle, and the left ventricular systolic pressure (LVSP) was measured.

The left ventricular hypertrophy was determined by measuring the ratio of left ventricular weight (LVW) (mg) to body weight (BW) (g) in the 19-week-old mice.

The test results are shown in FIGS. 5a to 5c and FIG. 6.

FIGS. 5a to 5c show hypertension symptoms in the TCTP-overexpressing transgenic mice. In the figures, Tg denotes transgenic mice, Lm denotes non-transgenic littermates as a control group, M denotes a male, and F denotes a female.

As shown in FIGS. 5a to 5c, the TCTP-overexpressing male and female transgenic mice showed more than 15-mmHg increases in the tail systolic blood pressure (T-SBP) as compared to the control group, indicating hypertension symptoms. Furthermore, the carotid systolic blood pressure (C-SBP) and the left ventricular systolic blood pressure (LVSP) were about 15-20 mmHg higher in the TCTP-overexpressing transgenic mice than those in the control group, and such hypertension symptoms were shown in more than 6-week-old, TCTP-overexpressing transgenic mice.

FIG. 6 shows left ventricular hypertrophy in 19-week-old transgenic mice used in the present invention, in which the left ventricular hypertrophy is expressed as the ratio of left ventricular weight (LWM) (mg) to body weight (BW) (g). In FIG. 6, reference numeral 1 in the horizontal axis denotes the control female mice, 2 denotes the transgenic female mice, 3 denotes the control male mice, and 4 denotes the transgenic male mice.

As shown in FIG. 6, both the male and female of the TCTP-overexpressing transgenic mice showed heart hypertrophy as compared to the control group.

As described above, as the composition used in the invention causes hypertension and heart hypertrophy, antihypertensive drug candidates can be developed by screening substances which react with the composition.

The TCTP-overexpressing transgenic mice show hypertension and heart hypertrophy as phenotypes, so that they will be useful in screening a test substance developed for the treatment and improvement of hypertension and heart hypertrophy, in which the screening comprises administering the test substance to the transgenic mice and observing the extent of treatment and improvement of the hypertension and heart hypertrophy.

### Industrial Applicability

The composition used in the invention has the effect of causing hypertension and heart hypertrophy.

Thus, the composition and the screening method using the same will be useful for the investigation and development of antihypertensive drugs.

Furthermore, the transgenic mice show hypertension or heart hypertrophy as a phenotype. Thus, the transgenic mice will be useful in screening test substances developed for the treatment and improvement of hypertension and heart hypertrophy. test substances developed for the treatment and improvement of hypertension and heart hypertrophy.

## Claims

1. Use of a composition which contains a mammalian TCTP gene for screening antihypertensive drugs, wherein the antihypertensive drugs inhibit an expression of the mammalian TCTP gene.

2. The use of Claim 1, wherein the TCTP gene has either a base sequence of SEQ ID NO: 1 or a base sequence having one or more disruption, deletion, insertion, point, substitution, nonsense, missense, polymorphism or rearrangement mutations in the base sequence of SEQ ID NO: 1.

3. Use of a composition which contains a mammalian TCTP protein for screening antihypertensive drugs, wherein the antihypertensive drugs inhibit the function of the mammalian TCTP protein to act as an intracellular inhibitor of the Na/K ATPase pump.

4. The use of Claim 3, wherein the TCTP protein has either an amino acid sequence of SEQ ID NO: 2 or is a polypeptide fragment which can be expressed from a gene of a base sequence having one or more disruption, deletion, insertion, point, substitution, nonsense, missense, polymorphism or rearrangement mutations in the base sequence of SEQ ID NO: 1 and shows physiological activity equal to that of TCTP.

5. A method for screening antihypertensive drugs, which uses a composition containing a mammalian TCTP gene or a mammalian TCTP gene which has either a base sequence of SEQ ID NO: 1 or a base sequence having one or more disruption, deletion, insertion, point, substitution, nonsense, missense, polymorphism or rearrangement mutations in the base sequence of SEQ ID NO: 1, as a target substance, wherein the antihypertensive drugs inhibit an expression of the mammalian TCTP gene.

6. The method of Claim 5, which comprises the steps of: contacting the composition with a test substance; and examining the reaction between the composition and the test substance so as to determine if the test substance shows the activity to inhibit the expression of the gene.

7. A method for screening antihypertensive drugs, which uses a composition containing a mammalian TCTP protein or a mammalian TCTP protein which has either an amino acid sequence of SEQ ID NO: 2 or is a polypeptide fragment which can be expressed from a gene of a base sequence having one or more disruption, deletion, insertion, point, substitution, nonsense, missense, polymorphism or rearrangement mutations in the base sequence of SEQ ID NO: 1 and shows physiological activity equal to that of TCTP as a target substance, wherein the antihypertensive drugs inhibit the function of the mammalian TCTP protein to act as an intracellular inhibitor of the Na/K ATPase pump.

8. The method of Claim 7, which comprises the steps of: contacting the composition with a test substance; and examining the reaction between the composition and the test substance so as to determine if the test substance shows the activity to inhibit the function of the mammalian TCTP protein to act as an intracellular inhibitor of the Na/K ATPase pump.

9. The method of Claims 6 or 8, which uses a method of measuring the activity of the TCTP gene or protein after reacting the TCTP gene or protein with the test substance; a yeast two-hybrid method; screening of a phage-displayed peptide clone binding to the TCTP protein; high throughput screening (HTS) using natural and chemical library; drug hit HTS; cell-based screening; or a screening method using a DNA array.

10. A method for screening antihypertensive drugs, which comprises the steps of:
administering test substances to transgenic mice; observing the extent of improvement of hypertension and heart hypertrophy symptoms in the animals; and screening test substances showing the improvement,
wherein the transgenic mice contain a mammalian TCTP gene at somatic and generative cells by the introduction of the mammalian TCTP gene at the embryonic stage, and thus phenotypes of hypertension and heart hypertrophy by the overexpression of a TCTP protein from the TCTP gene, and wherein the antihypertensive drugs inhibit an expression of the mammalian TCTP gene or the function of the mammalian TCTP protein to act as an intracellular inhibitor of the Na/K ATPase pump.

11. The method of claim 10, wherein the TCTP gene has either a base sequence of SEQ ID NO: 1 or a base sequence having one or more disruption, deletion, insertion, point, substitution, nonsense, missense, polymorphism or rearrangements mutation in the base sequence of SEQ ID NO: 1.

12. The method of claim 10, wherein the TCTP gene is inserted into a vector DNA containing a cytomegalovirus enhancer (CMV-IE), a chicken beta-actin promoter and a rabbit beta-globin poly A-tail.

## Patentansprüche

1. Verwendung einer Zusammensetzung, die ein Säuger-TCTP-Gen enthält, zum Screenen von Antihypertensiva, wobei die Antihypertensiva eine Expression des Säuger-TCTP-Gens inhibieren.

2. Verwendung nach Anspruch 1, wobei das TCTP-Gen entweder eine Basensequenz von SEQ ID NO: 1 oder eine Basensequenz mit ein oder mehr Disruptions-, Deletions-, Insertions-, Punkt-, Substitutions-, Nonsense-, Missense-, Polymorphismus- oder Umlagerungsmutationen in der Basensequenz von SEQ ID NO: 1 hat.

3. Verwendung einer Zusammensetzung, die ein Säuger-TCTP-Protein enthält, zum Screenen von Antihypertensiva, wobei die Antihypertensiva die Funktion des Säuger-TCTP-Proteins als intrazellulärer Inhibitor der Na/K-ATPase-Pumpe inhibieren.

4. Verwendung nach Anspruch 3, wobei das TCTP-Protein entweder eine Aminosäuresequenz von SEQ ID NO: 2 hat oder ein Polypeptidfragment ist, das von einem Gen mit einer Basensequenz mit ein oder mehr Disruptions-, Deletions-, Insertions-, Punkt-, Substitutions-, Nonsense-, Missense-, Polymorphismus- oder Umlagerungsmutationen in der Basensequenz von SEQ ID NO: 1 exprimiert wird und physiologische Aktivität gleich der von TCTP zeigt.

5. Verfahren zum Screenen von Antihypertensiva, welches eine Zusammensetzung als Zielsubstanz verwendet, die ein Säuger-TCTP-Gen enthält oder ein Säuger-TCTP-Gen, das entweder eine Basensequenz von SEQ ID NO: 1 oder eine Basensequenz mit ein oder mehr Disruptions-, Deletions-, Insertions-, Punkt-, Substitutions-, Nonsense-, Missense-, Polymorphismus- oder Umlagerungsmutationen in der Basensequenz von SEQ ID NO: 1 hat, wobei die Antihypertensiva eine Expression des Säuger-TCTP-Gens inhibieren.

6. Verfahren nach Anspruch 5, welches die Schritte umfasst: In Kontakt bringen der Zusammensetzung mit einer Testsubstanz; und Prüfen der Reaktion zwischen der Zusammensetzung und der Testsubstanz, um festzustellen, ob die Testsubstanz die Aktivität zeigt, die Expression des Gens zu inhibieren.

7. Verfahren zum Screenen von Antihypertensiva, welches als Zielsubstanz eine Zusammensetzung verwendet, die ein Säuger-TCTP-Protein enthält oder ein Säuger-TCTP-Protein, das entweder eine Aminosäuresequenz von SEQ ID NO: 2 hat oder ein Polypeptidfragment ist, das von einem Gen mit einer Basensequenz mit ein oder mehr Disruptions-, Deletions-, Insertions-, Punkt-, Substitutions-, Nonsense-, Missense-, Polymorphismus- oder Umlagerungsmutationen in der Basensequenz von SEQ ID NO: 1 exprimiert wird und physiologische Aktivität gleich der von TCTP zeigt, wobei die Antihypertensiva die Funktion des Säuger-TCTP-Proteins als intrazellulärer Inhibitor der Na/K-ATPase-Pumpe inhibieren.

8. Verfahren nach Anspruch 7, welches die Schritte umfasst: In Kontakt bringen der Zusammensetzung mit einer Testsubstanz; und Prüfen der Reaktion zwischen der Zusammensetzung und der Testsubstanz, um festzustellen, ob die Testsubstanz die Aktivität zeigt, die Funktion des Säuger-TCTP-Proteins als intrazellulärer Inhibitor der Na/K-ATPase-Pumpe inhibieren.

9. Verfahren nach Anspruch 6 oder 8, welches ein Verfahren zur Messung der Aktivität des TCTP-Gens oder -Proteins nach Reaktion des TCTP-Gens oder -Proteins mit der Testsubstanz; ein Hefe-Zwei-Hybrid-Verfahren; Screening eines an das TCTP-Protein bindenden Peptidklons im Phagen-Display; Hochdurchsatz-Screening mit natürlichen und chemischen Bibliotheken; Wirkstoff-Treffer-HTS; zellbasiertes Screening; oder ein Screening-Verfahren mit einem DNA-Array verwendet.

10. Verfahren zum Screenen von Antihypertensiva, welches die Schritte umfasst: Verabreichen von Testsubstanzen an transgene Mäuse; Beobachten des Maßes der Verbesserung von Hypertonie und Herzhypertrophiesymptomen an den Tieren; und Screening der Testsubstanzen, die die Verbesserung zeigen, wobei die transgenen Mäuse ein Säuger-TCTP-Gen in somatischen und generativen Zellen durch Einführen des Säuger-TCTP-Gens im Embryonalstadium enthalten und somit Phänotypen von Hypertonie und Herzhypertrophie durch Überexpression eines TCTP-Proteins von dem TCTP-Gen, und wobei die Antihypertensiva eine Expression des Säuger-TCTP-Gens oder die Funktion des Säuger-TCTP-Proteins als intrazellulärer Inhibitor der Na/K-ATPase-Pumpe inhibieren.

11. Verfahren nach Anspruch 10, wobei das TCTP-Gen entweder eine Basensequenz von SEQ ID NO: 1 oder eine Basensequenz mit ein oder mehr Disruptions-, Deletions-, Insertions-, Punkt-, Substitutions-, Nonsense-, Missense-, Polymorphismus- oder Umlagerungsmutationen in der Basensequenz von SEQ ID NO: 1 hat.

12. Verfahren nach Anspruch 10, wobei das TCTP-Gen in eine Vektor-DNA inseriert ist, die einen Cytomegalovirus-Enhancer (CMV-IE), einen Hühner-beta-Actin-Promotor und einen Kaninchen-beta-Globin-Poly(A)-Schwanz enthält.

## Revendications

1. Utilisation d'une composition qui contient un gène TCTP de mammifères pour le criblage de médicaments antihypertenseurs, les médicaments antihypertenseurs inhibant une expression du gène TCTP de mammifère.

2. Utilisation selon la revendication 1, dans laquelle le gène TCTP a soit une séquence de bases de SEQ ID NO : 1 soit une séquence de bases comportant une ou plusieurs mutations d'interruption, de délétion, d'insertion, ponctuelle, de substitution, non-sens, faux-sens, de polymorphisme ou de réarrangement dans la séquence de bases de SEQ ID NO : 1.

3. Utilisation d'une composition qui contient une protéine TCTP de mammifère pour le criblage de médicaments antihypertenseurs, les médicaments antihypertenseurs inhibant la fonction de la protéine TCTP de mammifère pour agir en tant qu'inhibiteurs intracellulaires de la pompe Na/K ATPase.

4. Utilisation selon la revendication 3, dans laquelle la protéine TCTP soit a une séquence d'acides aminés de SEQ ID NO : 2 soit est un fragment polypeptidique qui peut être exprimé à partir d'un gène d'une séquence de bases comportant une ou plusieurs mutations d'interruption, de délétion, d'insertion, ponctuelle, de substitution, non-sens, faux-sens, de polymorphisme ou de réarrangement dans la séquence de bases de SEQ ID NO : 1 et est présente une activité physiologique égale à celle de la TCTP.

5. Procédé de criblage de médicaments antihypertenseurs, qui utilise une composition contenant un gène TCTP de mammifère ou un gène TCTP de mammifère qui a soit une séquence de bases de SEQ ID NO : 1 soit une séquence de bases comportant une ou plusieurs mutations d'interruption, de délétion, d'insertion, ponctuelle, de substitution, non-sens, faux-sens, de polymorphisme ou de réarrangement dans la séquence de bases de SEQ ID NO : 1, en tant que substance cible, dans lequel les médicaments antihypertenseurs inhibent une expression du gène TCTP de mammifère.

6. Procédé selon la revendication 5, qui comprend les étapes suivantes : la mise en contact de la composition avec une substance à tester ; et l'examen de la réaction entre la composition et la substance à tester afin de déterminer si la substance à tester présente l'activité d'inhiber l'expression du gène.

7. Procédé de criblage de médicaments antihypertenseurs, qui utilise une composition contenant une protéine TCTP de mammifère ou une protéine TCTP de mammifère qui soit a une séquence d'acides aminés de SEQ ID NO : 2 soit est un fragment polypeptidique qui peut être exprimé à partir d'un gène d'une séquence de bases comportant une ou plusieurs mutations d'interruption, de délétion, d'insertion, ponctuelle, de substitution, non-sens, faux-sens, de polymorphisme ou de réarrangement dans la séquence de bases de SEQ ID NO : 1 est présente une activité physiologique égale à celle de la TCTP en tant que substance cible, dans lequel les médicaments antihypertenseurs inhibent la fonction de la protéine TCTP de mammifère pour agir en tant qu'inhibiteurs intracellulaires de la pompe Na/K ATPase.

8. Procédé selon la revendication 7, qui comprend les étapes suivantes : la mise en contact de la composition avec une substance à tester ; et l'examen de la réaction entre la composition et la substance à tester afin de déterminer si la substance à tester présente l'activité d'inhiber la fonction de la protéine TCTP de mammifère pour agir en tant qu'inhibiteurs intracellulaires de la pompe Na/K ATPase.

9. Procédé selon les revendications 6 ou 8, qui utilise un procédé de mesure de l'activité du gène ou de la protéine TCTP après avoir fait réagir le gène de la protéine TCTP avec la substance à tester ; un procédé à deux hybrides de levure ; le criblage d'un clone de peptide présenté par un phage se liant à la protéine TCTP ; un criblage à haut débit (HTS) utilisant une banque naturelle et chimique ; un HTS pour identifier des médicaments ; un criblage à base de cellules ; ou un procédé de criblage utilisant une puce à ADN.

10. Procédé de criblage de médicaments antihypertenseurs, qui comprend les étapes suivantes :
l'administration de substance à tester à des souris transgéniques ; l'observation de l'étendue de l'amélioration de l'hypertension et des symptômes d'hypertrophie cardiaque chez les animaux ; et le criblage des substances à tester présentant l'amélioration,
dans lequel les souris transgéniques contiennent un gène TCTP au niveau des cellules somatiques et reproductrices par l'introduction du gène TCTP de mammifère au stade embryonnaire, et ainsi les phénotypes de l'hypertension et de l'hypertrophie cardiaque par la surexpression d'une protéine TCTP à partir du gène TCTP, et dans lequel les médicaments antihypertenseurs inhibent une expression du gène TCTP de mammifère ou la fonction de la protéine TCTP de mammifère pour agir en tant qu'inhibiteur intracellulaire de la pompe Na/K ATPase.

11. Procédé selon la revendication 10, dans lequel le gène TCTP a soit une séquence de bases de SEQ ID NO : 1 ou une séquence de bases comportant une ou plusieurs mutations d'interruption, de délétion, d'insertion, ponctuelle, de substitution, non-sens, faux-sens, de polymorphisme ou de réarrangement dans la séquence de bases de SEQ ID NO : 1.

12. Procédé selon la revendication 10, dans lequel le gène TCTP est inséré dans un ADN de vecteur contenant un amplificateur du cytomégalovirus (CMV-IE), un promoteur de la bêta-actine du poulet et une queue poly A de bêta-globine de lapin.


### Sequence Listing

<110> LEE, Kyung lim
   OH, Goo-Taeg
   CHO, Myeong-Chan
   KIM, Min-Jeong
<120> Composition for screening anti-hypertension drug comprising mammal TCTP gene or its protein product, and method for screening anti-hypertension drug using said composition
<130> 04PP088
<150> KR 10-2003-0040519
   <151> 2003-06-21
<150> KR 10-2004-0043909
   <151> 2004-06-15
<160> 2
<170> KopatentIn 1.71
<210> 1
   <211> 858
   <212> DNA
   <213> Homo sapiens
<220>
   <221> mRNA
   <222> (1)..(858)
   <223> TCTP gene
<400> 1
   cgctcccccc tccccccgag cgccgctccg gctgcaccgc gctcgctccg agtttcaggc        60
   tcgtgctaag ctagcgccgt cgtcgtctcc cttcagtcgc catcatgatt atctaccggg       120
   acctcatcag ccacgatgag atgttctccg acatctacaa gatccgggag atcgcggacg       180
   ggttgtgcct ggaggtggag gggaagatgg tcagtaggac agaaggtaac attgatgact       240
   cgctcattgg tggaaatgcc tccgctgaag gccccgaggg cgaaggtacc gaaagcacag        300
   taatcactgg tgtcgatatt gtcatgaacc atcacctgca ggaaacaagt ttcacaaaag        360
   aagcctacaa gaagtacatc aaagattaca tgaaatcaat caaagggaaa cttgaagaac        420
   agagaccaga aagagtaaaa ccttttatga caggggctgc agaacaaatc aagcacatcc        480
   ttgctaattt caaaaactac cagttcttta ttggtgaaaa catgaatcca gatggcatgg        540
   ttgctctatt ggactaccgt gaggatggtg tgaccccata tatgattttc tttaaggatg        600
   gtttagaaat ggaaaaatgt taacaaatgt ggcaattatt ttggatctat cacctgtcat        660
   cataactggc ttctgcttgt catccacaca acaccaggac ttaagacaaa tgggactgat        720
   gtcatcttga gctcttcatt tattttgact gtgatttatt tggagtggag gcattgtttt        780
   taagaaaaac atgtcatgta ggttgtctaa aaataaaatg catttaaact caaaaaaaaa        840
   aaaaaaaaaa aaaaaaaa                                                      858
<210> 2
   <211> 172
   <212> PRT
   <213> Homo sapiens
<220>
   <221> CHAIN
   <222> (1)..(172)
   <223> TCTP protein
<400> 2
   Met Ile Ile Tyr Arg Asp Leu Ile Ser His Asp Glu Met Phe Ser Asp
   1               5                   10                  15
   Ile Tyr Lys Ile Arg Glu Ile Ala Asp Gly Leu Cys Leu Glu Val Glu
                   20                  25                  30
   Gly Lys Met Val Ser Arg Thr Glu Gly Asn Ile Asp Asp Ser Leu Ile
           35                  40                  45
   Gly Gly Asn Ala Ser Ala Glu Gly Pro Glu Gly Glu Gly Thr Glu Ser
       50                  55                  60
   Thr Val Ile Thr Gly Val Asp Ile Val Met Asn His His Leu Gln Glu
   65                  70                  75                  80
   Thr Ser Phe Thr Lys Glu Ala Tyr Lys Lys Tyr Ile Lys Asp Tyr Met
                   85                  90                  95
   Lys Ser Ile Lys Gly Lys Leu Glu Glu Gln Arg Pro Glu Arg Val Lys
               100                 105                 110
   Pro Phe Met Thr Gly Ala Ala Glu Gln Ile Lys His Ile Leu Ala Asn
           115                 120                 125
   Phe Lys Asn Tyr Gln Phe Phe Ile Gly Glu Asn Met Asn Pro Asp Gly
       130                 135                 140
   Met Val Ala Leu Leu Asp Tyr Arg Glu Asp Gly Val Thr Pro Tyr Met
   145                 150                 155                 160
   Ile Phe Phe Lys Asp Gly Leu Glu Met Glu Lys Cys
                   165                 170